# EUROPEAN PATENT APPLICATION

(11) **EP 4 434 569 A1**
(43) Date of publication of application: **25.09.2024**
(21) Application number: 22895511.8
(22) Date of filing: 09.11.2022
(51) Int. Cl.: A61M 25/095, A61J 15/00

(54) **IMAGING DEVICE**

(30) Priority: 16.11.2021 JP 2021186279
(71) Applicant: STANLEY ELECTRIC CO., LTD., Tokyo 153-8636 (JP); Met Co., Ltd., Tokyo 192-0081 (JP)
(72) Inventor: KAMIMURA, Yusuke, Tokyo 153-8636 (JP); MATOU, Takashi, Tokyo 1920081 (JP); TAMADA, Shinzo, Tokyo 1920081 (JP); EURA, Fumio, Tokyo 1920081 (JP)
(74) Representative: Schmidbauer, Andreas Konrad
(86) International application number: PCT/JP2022/041786
(87) International publication number: WO 2023/090229

(57) **Abstract**

To more easily verify a tube has reached a desired position in a target object.

An imaging apparatus including: a tube; a light-emitting part in the tube; a driver that switches emission and non-emission of light from the light emitting part; a camera that takes images of the target object; a controller that controls the driver and generates a display image using the image obtained from the camera; and a display that displays the display image; where the controller generates a light position image corresponding to the light from the light-emitting part based on the difference between a first image obtained from the camera correlated with the light emission and a second image obtained from the camera correlated with the light non-emission, and generates the display image by overlaying the light position image in a visible color on an exterior image of the target object obtained from the camera.

## Description

### TECHNICAL FIELD

The present disclosure relates to an imaging apparatus.

### BACKGROUND ART

International Publication No. 2018/207753 (Patent Document 1) discloses a catheter device provided with: a tube inserted into a human body; a light emitting part which is provided on a distal end side of the tube and emits a light for verifying the position of the tube; a power supply line which is provided along the tube and supplies power to the light emitting part; and a connector part provided on a proximal end side of the tube for connecting the power supply line installed in the tube and an external device that supplies power to the power supply line from outside. In this catheter device, when the distal end of the tube inserted into the human body reaches a desired position in the human body (for example, the stomach), light emitted from the light emitting part is transmitted through the human body and the light is received, and when the signal corresponding to the amount of the received light exceeds a preset value, a display on a control part will indicate that the distal end of the tube has reached the stomach. Further, International Publication No. 2020/136936 (Patent Document 2) discloses that a tube (catheter) is provided with a light emitting part not only at its distal end but also at the intermediate portion of the tube that enters the body.

### PRIOR ART DOCUMENT

### PATENT DOCUMENT

[Patent Document 1] International Publication No. 2018/207753
[Patent Document 2] International Publication No. 2020/136936

### SUMMARY OF THE INVENTION

### TECHNICAL PROBLEM

In a specific aspect, it is an object of the present disclosure to provide a technique which enables to more easily verify that a tube such as a catheter has reached a desired position in a target object.

### SOLUTION TO THE PROBLEM

An imaging apparatus according to one aspect of the present disclosure is an imaging apparatus including: (a) a tube used with at least one end side disposed inside or on the outside surface of a target object; (b) a light emitting part disposed in the tube; (c) a driver connected to the light emitting part and switches between a light emission and a light non-emission from the light emitting part; (d) a camera that takes an image of the target object; (e) a controller that is connected to each of the camera and the driver, controls the operation of the driver, and generates a display image using the image obtained from the camera; and (f) a display that is connected to the controller and displays the display image; (g) where the controller generates a light position image which corresponds to the light from the light emitting part based on the difference between a first image obtained from the camera correlated with the time of the light emission from the light emitting part and a second image obtained from the camera correlated with the time of the light non-emission from the light emitting part, and generates the display image by overlaying the light position image in a visible color on an exterior image of the target object obtained from the camera.

According to the above configuration, it is possible to more easily verify that a tube such as a catheter has reached a desired position in a target object.

### BRIEF DESCRIPTION OF THE DRAWINGS

FIG. 1 is a diagram for explaining the schematic configuration of an imaging apparatus according to one embodiment.
FIG. 2 is a diagram schematically showing an example of images captured by the imaging apparatus 1 and displayed on the display 24.
FIG. 3 is a block diagram for explaining the configuration related to information processing of the imaging apparatus 1.
FIG. 4A is a diagram schematically showing the configuration of the camera 20.
FIG. 4B is a diagram showing an example of a circuit configuration for realizing each pixel portion 40.
FIG. 5A is a potential diagram showing the state of distribution of electric charges.
FIG. 5B is a diagram schematically showing the process of taking the difference between the image obtained by electric charges "A" plus "C" (A+C) of the first electric charge storage part 46a and the image obtained by electric charges "B" (B) of the second electric charge storage part 46b.
FIG. 6 is a time chart showing the overall operation of the imaging apparatus 1.
FIG. 7 is a time chart showing the operation during period T1 which relates to capturing images by the camera shown in FIG. 6.
FIG. 8 is a time chart showing the operation during period T2 which relates to capturing images by the camera shown in FIG. 7.
FIG. 9 is a diagram schematically showing the overall operation of the imaging apparatus 1.

### MODE FOR CARRYING OUT THE INVENTION

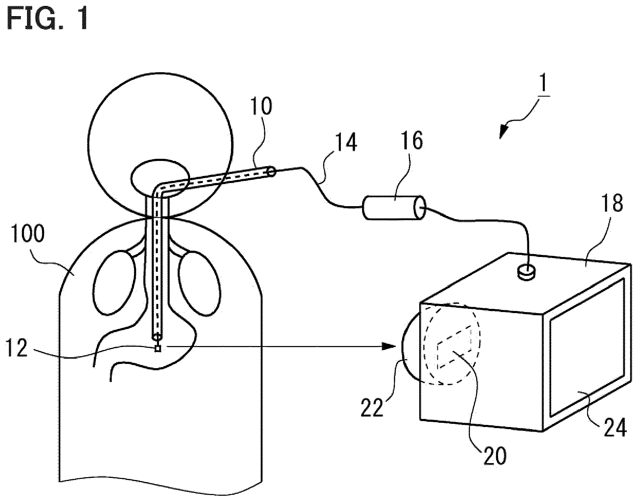

FIG. 1 is a diagram for explaining the schematic configuration of an imaging apparatus according to one embodiment. The imaging apparatus 1 of the present embodiment enables to easily verify that a distal end of a catheter 10 inserted into a human body 100 has reached a desired position (in the illustrated example, the position of the stomach) using an image displayed on a display 24.

The catheter 10 is a flexible long thin hollow tube made of a material that does not have a negative effect on a human body, and is used to deliver desired substances (e.g. nutrients, medicines, etc.) into the human body 100.

A light emitting diode (LED) 12 is disposed at one end of the catheter 10 and emits light of a wavelength that can pass through the human body 100. In this embodiment, for example, an infrared LED that emits infrared light with a wavelength of 850 nm is used. Further, in this embodiment, the LED 12 is placed at one end of the catheter 10, but when a stylet is inserted in the catheter 10, the LED 12 can be placed at the tip of the stylet which is inserted in the catheter 10. Furthermore, the LED 12 may be placed not only at one distal end of the catheter 10 and/or the stylet which is inserted into the catheter 10, but also a plurality of LEDs may be arranged apart at the catheter 10 placed inside or on the outside surface of the human body 100 and/or the stylet which is inserted in the catheter 10. In this case, the plurality of LEDs 12 may consist of light sources of the same or different wavelengths.

A wiring cable (signal line) 14 is arranged through the inside of the catheter 10 and electrically connects the LED 12 and a driver 16. This wiring cable 14 is for supplying driving voltage from the driver 16 to the LED 12.

The driver (light source control circuit) 16 operates under the control of a controller 18, and switches the LED 12 between light-on and light-off (i.e., light emission and light non-emission) by supplying a drive voltage to the LED 12.

The controller 18 photographs the light which is emitted from the LED 12 and transmitted through the human body 100, photographs the exterior of the human body 100, and generates a display image that shows the state of the photographed results. This imaging apparatus 18 includes a camera (imaging element) 20 for photographing the human body 100 and the light emitted from the LED 12, and a lens 22 arranged on the light receiving surface side of the camera 20.

The display 24 is connected to the controller 18 and displays the display image generated by the controller 18. As for the display 24, various known displays such as a liquid crystal display, an organic EL display, and a cathode ray tube display can be used.

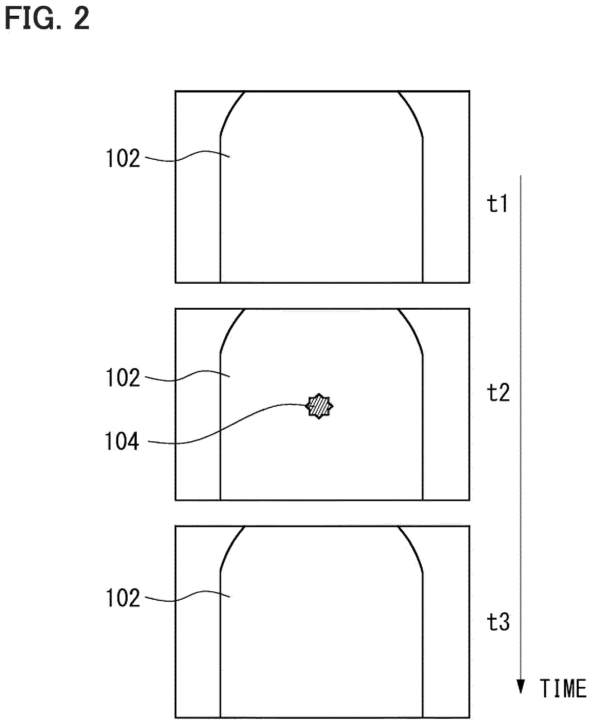

FIG. 2 is a diagram schematically showing an example of images captured by the imaging apparatus 1 and displayed on the display 24. For example, at time t1 and t3, the human body 100 is illuminated by surrounding environmental light (including external illumination such as strobe light), and a human body image (exterior image) 102 is displayed on the display 24 of the imaging apparatus 1. Further, for example, at time t2, the light from the LED 12 that has passed through the human body 100 is extracted, and a light position image 104 corresponding to the light from the LED is overlaid on the human body image 102 and displayed. The light position image 104 can be displayed on the display 24, and is an image of an appropriate color visible to humans. By displaying such an image, it is possible to easily verify where the LED 12 placed at one distal end side of the catheter 10 is located in the human body 100. Further, in an embodiment in which a plurality of LEDs 12 are arranged spaced apart at the distal end and/or intermediate position of the catheter 10, it is possible to easily verify where the plurality of LEDs 12 arranged spaced apart at the distal end and/or intermediate position of the catheter 10 are located in the human body 100.

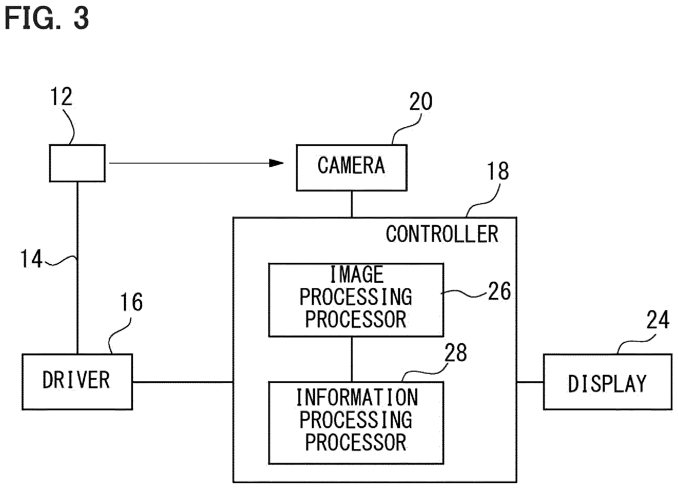

FIG. 3 is a block diagram for explaining the configuration related to information processing of the imaging apparatus 1. The imaging apparatus 1 includes the above-described LED 12, the wiring cable 14, the driver 16, the controller 18, the camera 20, and the display 24. The controller 18 is configured to include an image processing processor 26 that performs predetermined image processing on the image captured by the camera 20, and an information processing processor 28 that controls the display 24 to display an image (a display image) obtained by image processing performed by the image processing processor 26, and also controls the operation of the driver 16. Details of the operations by the image processing processor 26 and the information processing processor 28 (information processing) will be described later.

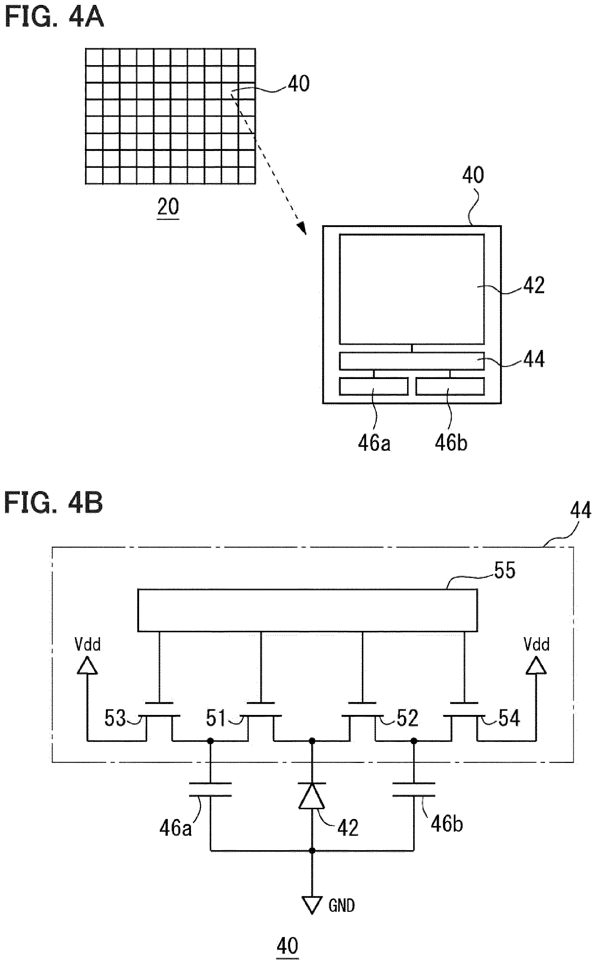

FIG. 4A is a diagram schematically showing the configuration of the camera 20. As illustrated in the figure, the camera 20 includes a plurality of regularly arranged pixel portions 40. Each pixel portion 40 includes a photodiode 42 which is a photoelectric conversion element that converts incident light into electric charges, an electric charge distribution circuit 44 that distributes the electric charges generated by the photodiode 42, a first electric charge storage part 46a and a second electric charge storage part 46b that stores the electric charges distributed by the electric charge distribution circuit 44.

FIG. 4B is a diagram showing an example of a circuit configuration for realizing each pixel portion 40. Each of the first electric charge storage part 46a and the second electric charge storage part 46b is, for example, a capacitive element, and each is connected in parallel to the photodiode 42. The anode of the photodiode 42 and one end of each of the first electric charge storage part 46a and the second electric charge storage part 46b are connected to a reference potential terminal (GND terminal).

The electric charge distribution circuit 44 is configured to include four field effect transistors 51, 52, 53, and 54, and a gate control circuit 55 that applies a control voltage to the gates (control terminals) of these field effect transistors 51 to 54. In the field effect transistor 51, one of its source/drain is connected to the other end of the first electric charge storage part 46a, and the other of its source/drain is connected to the cathode of the photodiode 42. In the field effect transistor 52, one of its source/drain is connected to the other end of the second electric charge storage part 46b, and the other of its source/drain is connected to the cathode of the photodiode 42. In the field effect transistor 53, one of its source/drain is connected to the other end of the first electric charge storage part 46a, and the other of its source/drain is connected to the power supply Vdd. In the field effect transistor 54, one of its source/drain is connected to the other end of the second electric charge storage part 46b, and the other of its source/drain is connected to the power supply Vdd.

When the field effect transistor 51 is turned on (conducting state) and the other field effect transistors 52 to 54 are turned off (non-conducting state) by the gate control circuit 55 whose operation is controlled by the information processing processor 28, the electric charges generated by the light received by photodiode 42 is stored in the first electric charge storage part 46a. Further, when the field effect transistor 52 is turned on (conducting state) and the other field effect transistors 51, 53 and 54 are turned off (non-conducting state) by the gate control circuit 55, the electric charges generated by the light received by photodiode 42 is stored in the second electric charge storage part 46b. Further, when the field effect transistor 53 is turned on and the other field effect transistors 51, 52, and 54 are turned off, the electric charges in the first electric charge storage part 46a is reset. Similarly, when the field effect transistor 54 is turned on and the other field effect transistors 51, 52, and 53 are turned off, the electric charges in the second electric charge storage part 46b is reset.

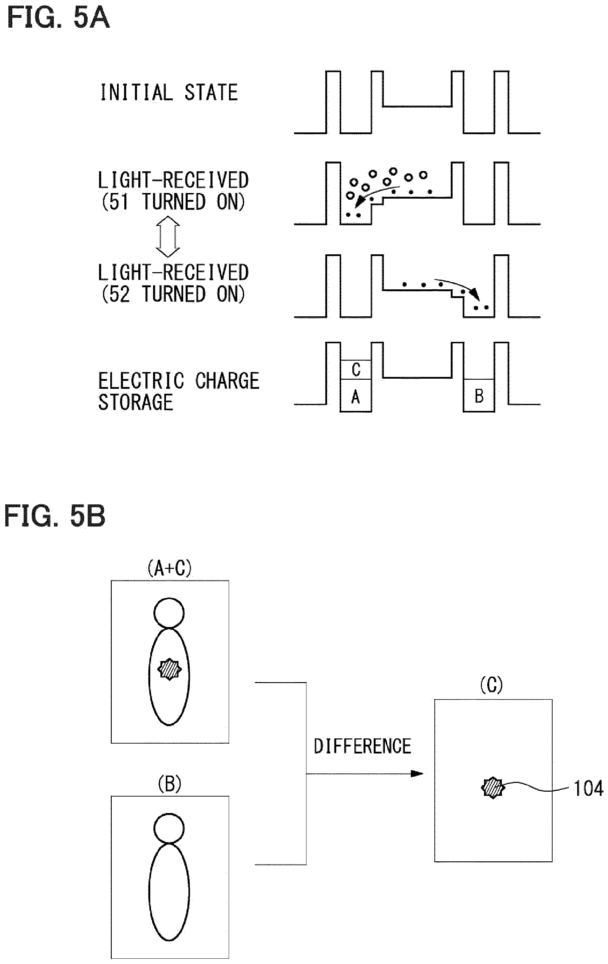

By alternately turning on the field effect transistors 51 and 52, the electric charges can be distributed and stored alternately in the first electric charge storage part 46a and the second electric charge storage part 46b. This state of distribution of electric charges is shown in a potential diagram in FIG. 5A. For example, when the LED 12 is turned on (light emission) at a timing when the field effect transistor 51 is turned on and the LED 12 is turned off at a timing when the field effect transistor 52 is turned on, the electric charges generated by the light emission of the LED 12 is stored in the first electric charge storage part 46a but not stored in the second electric charge storage part 46b. In FIG. 5A, the electric charges corresponding to the light emission of the LED 12 is denoted as "C". And in FIG. 5A, among the electric charges corresponding to the light generated when natural light or illumination light is reflected by the human body 100, the electric charges stored in the first electric charge storage part 46a is denoted as "A", and the electric charges stored in the second electric charge storage part 46b is denoted as as "B". Normally, electric charges A and electric charges B are approximately equal, therefore, as schematically shown in FIG. 5B, by taking the difference between the image obtained by electric charges "A" plus "C" (A+C) of the first electric charge storage part 46a and the image obtained by electric charges "B" (B) of the second electric charge storage part 46b, the light position image 104 caused by the light emission from the LED 12 can be generated.

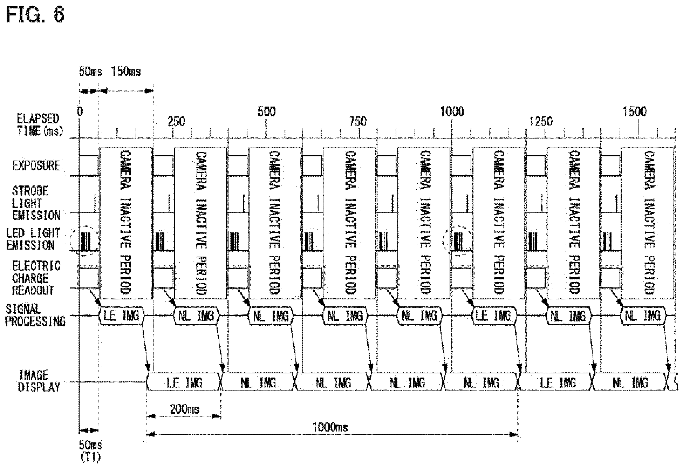

FIG. 6 is a time chart showing the overall operation of the imaging apparatus 1. The imaging apparatus 1 of this embodiment captures images at a cycle of 200 ms as an example. During the first 50 ms of this one cycle, operations related to capturing images by the camera 20, specifically, exposure, strobe light emission, LED light emission, and electric charge readout from the camera are executed. Further, during the latter 150 ms of this one cycle, the camera 20 stops operating in order to save power, and during that time, the controller 18 executes signal processing using the electric charges read out from the camera 20 and a display image is generated. This generated display image is displayed on the display 24 for 200 ms at a timing that overlaps with the next cycle. The display images include a "light emission image" (abbreviated as "LE IMG" in FIG. 6) which is an image in which the human body image 102 and the light position image 104 are overlaid, and a "normal image" (abbreviated as "NL IMG" in FIG. 6) which is an image that includes the human body image 102 but does not include the light position image 104. In this embodiment, one light emission image and four normal images are repeatedly displayed on the display 24 in order, at 200 ms intervals every 1000 ms (1 sec).

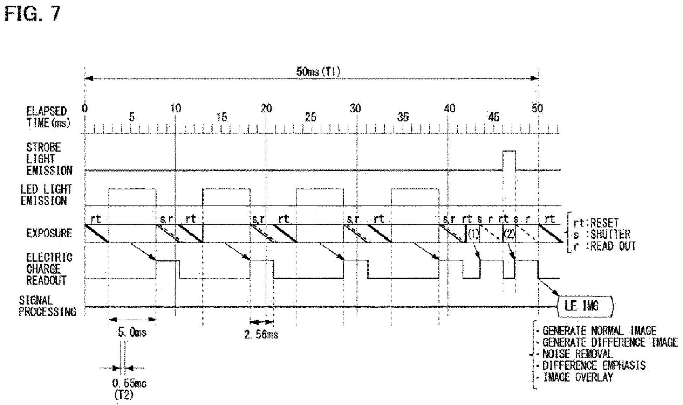

FIG. 7 is a time chart showing the operation during period T1 which relates to capturing images by the camera shown in FIG. 6. Here, a time chart of the operation related to generation of a light emission image is shown. The imaging apparatus 1 controls the driver 16 using the information processing processor 28 to cause the LED 12 to emit light and causes the camera 20 to execute an exposure operation. In this embodiment, this operation is executed a predetermined number of times (four times as an example). Before the LED 12 emits light each time, electric charges in the first electric charge storage part 46a and the second electric charge storage part 46b which are included in each pixel portion 40 of the camera 20 are reset, and then the shutter of the camera 20 is opened and exposure of each pixel portion 40 is executed. Further, after the LED 12 emits light, the shutter of the camera 20 is closed and the electric charges stored in each of the first electric charge storage part 46a and the second electric charge storage part 46b are read out. The light emitting time of the LED 12 is, for example, 5.0 ms as shown in the figure, and the LED 12 emits light intermittently (that is, it alternately turns on and off) during this time. Further, the time required to read out the electric charges is, for example, 2.56 ms.

A difference image is generated by the image processing processor 26 based on each of the electric charges of the first electric charge storage part 46a and the second electric charge storage part 46b obtained by the above four imaging operations. The "difference image" here is an image which corresponds to the light position image 104 described above. In this embodiment, the difference image is obtained by taking the difference between the image based on the electric charges of the first electric charge storage part 46a and the image based on the electric charges of the second electric charge storage part 46b, and the light position image 104 is obtained by further performing image processing such as noise removal and difference emphasis on the difference image, and further processing such as conversion to data representing color tones within the human visible range. Here, note that a color tone-converted difference image may be used as it is as the light position image 104, in principle.

Further, after the above-described four imaging operations, a strobe (not shown) of the camera 20 is used, and by using (i) the electric charges stored in each of the first electric charge storage part 46a and the second electric charge storage part 46b while not causing the camera 20 to expose light during a period (1) in which the strobe does not emit light and (ii) the electric charges stored in each of the first electric charge storage part 46a and the second electric charge storage part 46b while causing the camera 20 to expose light during a period (2) in which the strobe does emit light, the image processing processor 26 obtains the difference between these electric charges, thereby a normal image is generated. Here again, reset is performed before the camera 20 is caused to expose light, and then the shutter of the camera 20 is opened to execute the exposure, and then after the shutter is closed, electric charges are read out. Then, the image processor 26 generates a light emission image by overlaying the light position image on the normal image. Here, although it is possible to obtain a clearer normal image with the offset component removed by obtaining the difference as described above, the normal image can be generated by omitting such processing, and use the electric charges corresponding to period (2) during which the strobe emits light.

Here, note that generation of the "normal image" shown in FIG. 6 is also performed in the same manner as described above. For example, the light emission of the LED 12 and generation of the difference image based on the light emission are not executed, and as in the case shown in FIG. 7, the normal image is generated in the same manner as described above from roughly after 42 ms have elapsed within period T1.

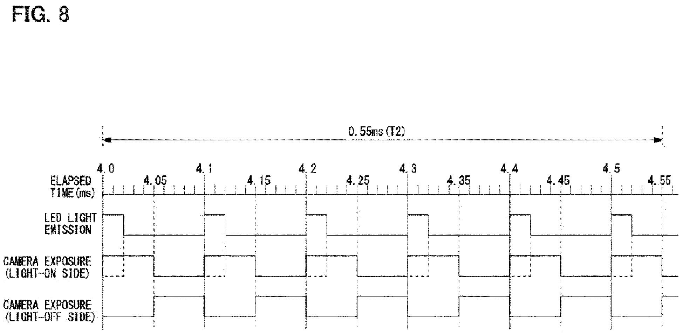

FIG. 8 is a time chart showing the operation during period T2 which relates to capturing images by the camera shown in FIG. 7. Here, a detailed time chart of the operation related to generation of a light emission image is shown. Here, period T2 indicates a period of 0.55 ms, which is part of the above-described period T1. The operation shown here is repeated within period T1.

As shown in the figure, the LED 12 is controlled by the driver 16 to repeat the operation of turning on for 0.02 ms and then turning off for 0.08 ms. Camera exposure (light-on side) represents a period during which electric charges are distributed and stored in the first electric charge storage part 46a, and camera exposure (light-off side) represents a period during which electric charges are distributed and stored in the second electric charge storage part 46b. As shown in the figure, these periods alternate every 0.05 ms. The light emission (light-on) timing of the LED 12 is associated with the period during which charges are stored in the first electric charge storage part 46a. Here, the LED 12 is controlled to emit light during the first 0.02 ms period of the period in which charges are stored in the first electric charge storage part 46a, and to turn off during the subsequent 0.03 ms period. In other words, since a certain period of time elapses even after the LED 12 has been switched from being turned on (light emission) to being turned off (light non-emission), electric charges will continue to be distributed to the first electric charge storage part 46a, and after the certain period of time has elapsed, electric charges will be distributed to the second electric charge storage part 46b. This is performed in consideration of the time required for photoelectric conversion in the photodiode 42, and specifically to prevent electric charges generated by light emission from the LED 12 from being distributed to the second electric charge storage part 46b.

After the above-described operation is repeated within a period of 5.0 ms, read out of the electric charges is initiated (refer to FIG. 7 described above). That is, electric charge storage operation (light exposure operation) to the first electric charge storage part 46a and electric charge storage operation (light exposure operation) to the second electric charge storage part 46b are repeatedly performed once every 0.1 ms within the period of 5.0 ms. Then, the electric charges obtained by such operations are read out, and one differential image is generated based on the electric charges. In detail, one difference image is obtained by taking the difference between an image obtained based on the electric charges of the first electric charge storage part 46a and an image obtained based on the electric charges of the second electric charge storage part 46b. Then, a light position image 104 is obtained using this difference image.

By distributing electric charges to the first electric charge storage part 46a and the second electric charge storage part 46b many times in a short period of time (5.0 ms in this embodiment), the electric charges caused by external light stored in the first electric charge storage part 46a and the electric charges caused by external light stored in the second electric charge storage part 46b are respectively averaged, and the difference between the two can be reduced. Further, since the LED 12 is turned on in accordance with the period of distribution of electric charges to the first electric charge storage part 46a and turned off in accordance with the period of distribution of electric charges to the second electric charge storage part 46b, in principle, electric charges caused by lighting of the LED 12 is stored only in the first electric charge storage part 46a. Therefore, in the difference image generated based on electric charges of the first electric charge storage part 46a and the second electric charge storage part 46b, the effect of noise due to external light and motion artifact (motion artifact is blurring of an image due to subtle body movement of a subject) can be significantly reduced. For example, it is possible to significantly reduce fluctuations in external light due to flickering of lights, swinging of curtains, etc., as well as fluctuations in light due to camera shake, subject shake, etc.

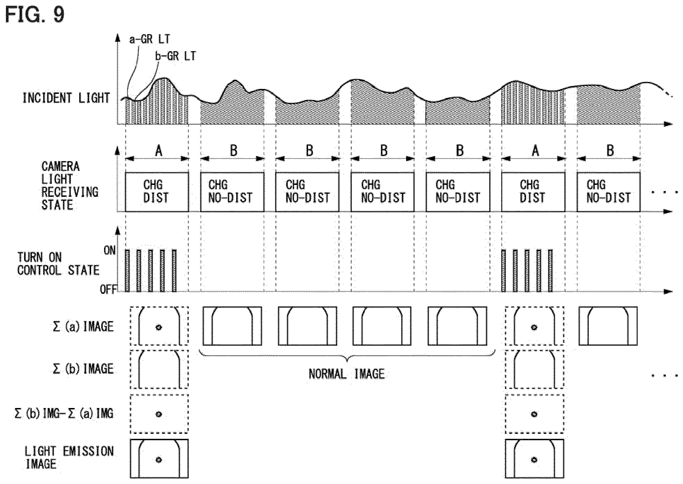

FIG. 9 is a diagram schematically showing the overall operation of the imaging apparatus 1. As shown in the upper part of the figure, most of the light incident on the camera 20 consists of external light (natural light and illumination light) other than light from the LED 12, and light intensity thereof can vary. Here, the illustrated a-group light (abbreviated as "a-GR LT" in FIG. 9) is the light received by the photodiode 42 in each pixel portion 40 of the camera 20 when the LED 12 emits light. The electric charges caused by this a-group light is stored in the first electric charge storage part 46a of each pixel portion 40. Further, the illustrated b-group light (abbreviated as "b-GR LT" in FIG. 9) is the light received by the photodiode 42 in each pixel portion 40 of the camera 20 when the LED 12 does not emit light. The electric charges caused by this b-group light is stored in the second electric charge storage part 46b of each pixel portion 40.

As shown in the second row of the figure, one cycle of light receiving state of the camera 20 consists of one period A during which electric charges are distributed (abbreviated as "CHG DIST" in FIG. 9) and four periods B during which electric charges are not distributed (abbreviated as "CHG NO-DIST" in FIG. 9). As shown in the third row in the figure, the LED 12 is turned on and off in accordance with a predetermined modulation frequency during period A under the control of the driver 16. This modulation frequency can be set, for example, within a range of about 1 kHz to 100 MHz, and is set to 10 kHz in this embodiment.

As shown in the third row of the figure, one image is generated by the image processing processor 26 using electric charges stored in the first electric charge storage part 46a by a-group light during period A. This is represented as ∑(a) image in the figure. Further, one image is generated by the image processing processor 26 using electric charges stored in the second electric charge storage part 46b by b-group light during period A. This is represented as ∑(b) image in the figure. By taking the difference between ∑(a) image and ∑(b) image (∑(b) image - ∑(a) image) and performing appropriate image processing, the light position image 104 described above is obtained. Further, by overlapping this light position image 104 and the human body image 102, the light emission image is obtained and is displayed on the display 24 by the information processing processor 28.

On the other hand, in each period B, without distributing electric charges, a normal image is generated by the image processing processor 26 by using electric charges stored in the first electric charge storage part 46a and the second electric charge storage part 46b while keeping the LED 12 turned off, and then the normal image is displayed on the display 24 by the information processing processor 28. As described above, one light emission image and four normal images are repeatedly displayed on the display 24 in sequence at 200 ms intervals. As a result, a user viewing the display 24 can perceive the light of the LED 12 as blinking while overlapping the human body. With such images on the display, the position of the LED 12, in other words, the position of distal end side of the catheter 10 can be easily verified. Further, in an embodiment in which a plurality of LEDs 12 are arranged at a distance at one distal end and/or an intermediate position of the catheter 10, the one distal end and/or the intermediate position of the catheter 10 can be easily verified. Here, note that depending on the switching cycle of one light emission image and four normal images, the user can perceive that the light emission image is substantially continuously displayed.

According to the embodiment described above, it is possible to more easily verify that the catheter has reached a desired position in a human body. For example, the change of the position of the LED 12 disposed on the catheter 10 from its original position due to peristaltic movement, reflux, contraction movement, or the like can be verified through the display 24. Therefore, incorrect insertion of the catheter into an unintended position can be prevented.

Note that the present disclosure is not limited to the content of the embodiment described above, and can be implemented with various modifications within the scope of the gist of the present disclosure. For example, although an LED has been cited as an example of the light emitting part, the light emitting part may be configured using a light emitting element other than an LED (for example, a laser element). Further, the light emitting part may be configured by guiding light from a light emitting element or the like to one end of the catheter using a light guide means such as an optical fiber. Further, the wavelength of the light emitted from the light emitting part is not limited to the above-described wavelength of infrared light as long as it can pass through the target object, and depending on the application, the wavelength of the emitted light may be a wavelength of ultraviolet light or a wavelength of visible light. Further, the camera 20 can also be provided with a plurality of wavelength filters so as to be able to detect a plurality of wavelengths of light emitted from the light emitting part. Further, a plurality of cameras 20 can be installed at a distance.

Further, in the embodiment described above, a photodiode is used as an example of a photoelectric conversion element, but the disclosure is not limited thereto. For example, an avalanche photodiode, a CCD sensor, a CMOS sensor, or the like may be used as a photoelectric conversion element.

Further, in the above-described embodiment, a human body has been cited as an example of a target object, and a case where a light emitting part is displaced inside the human body has been described. Further, the present disclosure is also applicable to, for example, abdominal cavity in the body, subcutaneous, intracranial, tissues including thoracic cavity, inside a canal from a mouth to an anus, inside of blood vessels including cerebral blood vessels, ureter, bladder, and the like. Further, the light emitting part may be arranged near the human body, such as on the outer surface of the human body. Further, the target object may be something other than a human body. Furthermore, if the target object is something other than a human body, a tube of material and shape suitable for that purpose may be used, and a catheter (optimized for a human body) need not necessarily be used.

Further, in the embodiment described above, an image processing processor and an information processing processor are separately described as an example of processors, but a processor that performs both image processing and information processing may also be used. Further, all or part of the functions of the image processing processor and the information processing processor may be provided on the camera side.

### REFERENCE SIGNS LIST

10: Catheter (Tube)
12: LED (Light emitting part)
14: Wiring cable (Signal line)
16: Driver (Light source control circuit)
18: Controller
20: Camera (Imaging element)
22:Lens
24:Display
26:Image processing processor
28:Information processing processor
40:Pixel portion
42:Photodiode
44:Electric charge distribution circuit
46a:First electric charge storage part
46b:Second electric charge storage part
100: Human body
102: Human body image (Exterior image)
104: Light position image

## Claims

1. An imaging apparatus comprising:
a tube used with at least one end side disposed inside or on the outside surface of a target object;
at least one light emitting part disposed in the tube;
a driver connected to the light emitting part and switches emission and non-emission of light from the light emitting part;
a camera that takes an image of the target object;
a controller that is connected to each of the camera and the driver, controls the operation of the driver, and generates a display image using the image obtained from the camera; and
a display that is connected to the controller and displays the display image;
wherein the controller generates a light position image which corresponds to the light from the light emitting part based on the difference between a first image obtained from the camera correlated with the time of the light emission from the light emitting part and a second image obtained from the camera correlated with the time of the light non-emission from the light emitting part, and generates the display image by overlaying the light position image in a visible color on an exterior image of the target object obtained from the camera.

2. An imaging apparatus comprising:
a tube used with at least one end side disposed inside or on the outside surface of a target object;
a stylet inserted in the tube;
at least one light emitting part disposed in the stylet;
a driver connected to the light emitting part and switches emission and non-emission of light from the light emitting part;
a camera that takes an image of the target object;
a controller that is connected to each of the camera and the driver, controls the operation of the driver, and generates a display image using the image obtained from the camera; and
a display that is connected to the controller and displays the display image;
wherein the controller generates a light position image which corresponds to the light from the light emitting part based on the difference between a first image obtained from the camera correlated with the time of the light emission from the light emitting part and a second image obtained from the camera correlated with the time of the light non-emission from the light emitting part, and generates the display image by overlaying the light position image in a visible color on an exterior image of the target object obtained from the camera.

3. An imaging apparatus comprising:
a tube used with at least one end side disposed inside or on the outside surface of a target object;
a stylet inserted in the tube;
at least one light emitting part disposed in the tube and the stylet;
a driver connected to the light emitting part and switches emission and non-emission of light from the light emitting part;
a camera that takes an image of the target object;
a controller that is connected to each of the camera and the driver, controls the operation of the driver, and generates a display image using the image obtained from the camera; and
a display that is connected to the controller and displays the display image;
wherein the controller generates a light position image which corresponds to the light from the light emitting part based on the difference between a first image obtained from the camera correlated with the time of the light emission from the light emitting part and a second image obtained from the camera correlated with the time of the light non-emission from the light emitting part, and generates the display image by overlaying the light position image in a visible color on an exterior image of the target object obtained from the camera.

4. The imaging apparatus according to any one of claims 1 to 3,
wherein the camera includes a plurality of pixel portions,
wherein each of the plurality of pixel portions includes a photoelectric conversion element, a first electric charge storage part, a second electric charge storage part, and an electric charge distribution circuit which causes to distribute and store electric charges generated by the photoelectric conversion element to the first electric charge storage part or the second electric charge storage part,
wherein the electric charge distribution circuit causes the first electric charge storage part to store electric charges generated by the photoelectric conversion element in correspondence with the light emission from the light emitting part, and causes the second electric charge storage part to store electric charges generated by the photoelectric conversion element in correspondence with the light non-emission from the light emitting part, and
wherein the controller obtains the first image based on the electric charges stored in the first electric charge storage part of each of the plurality of pixel portions, and obtains the second image based on the electric charges stored in the second electric charge storage part of each of the plurality of pixel portions.

5. The imaging apparatus according to claim 4,
wherein the driver switches the light emission and the light non-emission from the light emitting part according to a predetermined modulation frequency,
wherein the electric charge distribution circuit distributes electric charges to the first electric charge storage part or the second electric charge storage part corresponding to the switching according to the predetermined modulation frequency by the driver, and
wherein, after the electric charges are distributed by the electric charge distribution circuit a plurality of times, the controller obtains the first image based on the electric charges of the first electric charge storage part and obtains the second image based on the electric charges of the second electric charge storage part.

6. The imaging apparatus according to claim 5,
wherein, after the light emitting part switches from the light emission to the light non-emission, the electric charge distribution circuit distributes electric charges generated by the photoelectric conversion element to the first electric charge storage part until a certain period of time has elapsed, and distributes electric charges generated by the photoelectric conversion element to the second electric charge storage part after the certain period of time has elapsed.

7. The imaging apparatus according to claim 5 or 6,
wherein the modulation frequency is set in a range of 1 kHz or more and 100 MHz or less.

8. The imaging apparatus according to any one of claims 1 to 7,
wherein the light emitting part is a light emitting element connected to the driver via a signal line.

9. The imaging apparatus according to any one of claims 1 to 8,
wherein the target object is a human body, and
wherein the light emitted from the light emitting part has a wavelength that can pass through the human body.

10. The imaging apparatus according to claim 9,
wherein the tube is a catheter in which at least the one end side is disposed inside the human body.
